# EUROPÄISCHE PATENTSCHRIFT

(11) **EP 3 155 973 B1**
(45) Veröffentlichungstag und Bekanntmachung des Hinweises auf die Patenterteilung: **20.03.2019**
(21) Anmeldenummer: 16192977.3
(22) Anmeldetag: 10.10.2016
(51) Int. Cl.: A61B 10/06, A61B 10/04, A61B 17/34, A61B 90/00

(54) **BIOPSIEZANGE**
BIOPSY FORCEPS
PINCE À BIOPSIE

(30) Priorität: 16.10.2015 DE 202015105488 U
(43) Veröffentlichungstag der Anmeldung: 19.04.2017
(73) Patentinhaber: MTW-Endoskopie W. Haag KG, 46487 Wesel (DE)
(72) Erfinder: Haag, Wolfgang, 46487 Wesel (DE)
(74) Vertreter: Brötz, Helmut

(56) Entgegenhaltungen:
- JP-A- H10 276 967
- JP-A- 2002 224 121
- US-A1- 2009 099 414
- US-A1- 2010 312 141
- US-A1- 2012 123 201

## Beschreibung

### Gebiet der Technik

Die vorliegende Erfindung betrifft eine Biopsiezange, die insbesondere als Punktionsbiopsiezange ausgebildet sein kann. Bei einer gattungsgemäßen Biopsiezange handelt es sich um endoskopisches Zubehör. Sie umfasst ein längliches Zug-/Schubteil, an dessen distalem Längsende ein Zangenkopf angebracht ist und an dessen proximalem Ende sich eine Handhabungseinrichtung befindet, mittels welcher von einem Operateur der Zangenkopf bedarfsweise geöffnet und geschlossen werden kann. Die Biopsiezange kann mit zunächst geschlossenem Zangenkopf durch einen Arbeitskanal eines in einen menschlichen oder tierischen Körper eingeführten Endoskops geführt werden, so dass ihr distales Längsende über die Endoskopspitze hinausragt, wobei sich die Handhabungseinrichtung außerhalb des proximalen Endoskopendes und außerhalb des menschlichen bzw. tierischen Körpers befindet. Bei dieser Anordnung kann das aus den beiden Branchen des Zangenkopfs gebildete Zangenmaul mittels der Handhabungseinrichtung geöffnet werden, mittels den Branchen Gewebe ergriffen, die Branchen anschließend wieder zusammengedrückt und zwischen ihnen festgeklemmtes Gewebe abgezupft werden, um dieses, bspw. für Untersuchungen, aus dem Körper zu entfernen.

Gemäß dem Oberbegriff von Anspruch 1 betrifft die vorliegende Erfindung eine Biopsiezange, insbesondere Punktionsbiopsiezange, wobei die Biopsiezange einen Zangenkopf umfasst, wobei die Biopsiezange ein erstes Zug-/Schubteil umfasst, an dessen distalem Längsende der Zangenkopf angebracht ist, wobei die Biopsiezange eine flexible Ummantelung umfasst, die einen Hohlkanal aufweist, durch den das erste Zug-/Schubteil relativ zu der flexiblen Ummantelung längsverschieblich hindurchgeführt ist, wobei die Biopsiezange eine Handhabungseinrichtung umfasst, die eine Halteeinrichtung und eine erste Betätigungseinrichtung aufweist, wobei die Halteeinrichtung und die erste Betätigungseinrichtung gemeinsam eine erste Längsführung bilden, mittels der die erste Betätigungseinrichtung an der Halteeinrichtung relativ zu ihr entlang einer Betätigungsrichtung verschiebbar geführt ist, wobei die flexible Ummantelung, insbesondere an ihrem proximalen Längsende, mit der Halteeinrichtung verbunden ist, wobei das erste Zug-/Schubteil, insbesondere an seinem proximalen Längsende, mit der ersten Betätigungseinrichtung verbunden ist, wobei die Biopsiezange eine Wegbegrenzungseinrichtung umfasst, die an der ersten Längsführung an in Bezug auf die genannte Betätigungsrichtung wählbaren Positionen lösbar befestigbar ist und die in befestigtem Zustand einen Anschlag bildet, der einen Verschiebeweg der ersten Betätigungseinrichtung relativ zu der Halteeinrichtung in distaler Richtung begrenzt, wobei die Halteeinrichtung einen Führungskern aufweist, wobei die erste Betätigungsrichtung eine Führungshülse aufweist, wobei der Führungskern und die Führungshülse gemeinsam die erste Längsführung ausbilden, wobei die Wegbegrenzungseinrichtung eine Hülse aufweist, die auf den Führungskern aufgeschoben ist, wobei die Hülse in ihrer Wandung eine Gewindebohrung aufweist, in die eine Klemmschraube eingeschraubt ist, wobei die Hülse bei gelöster Klemmschraube auf dem Führungskern verschiebbar ist und wobei die Hülse bei festgezogener Klemmschraube an dem Führungskern fixiert ist.

### Stand der Technik

Im Stand der Technik sind Biopsiezangen bekannt, bei denen ein Zug-/Schubteil, an dessen distalem Längsende der Zangenkopf gehalten ist, in einem biegsamen Metallmantel längsverschieblich geführt ist. Während des Einführens in den Arbeitskanal des Endoskops ist der Zangenkopf in den Metallmantel zurückgezogen und darin geschützt. Wenn der Metallmantel aus dem Arbeitskanal nach distal hervortritt, wird das Zug-/Schubteil mittels der Handhabungseinrichtung nach distal aus dem Metallmantel hinausgeschoben, um zu dem relevanten Gewebe vorzudringen. Bei sog. Punktionsbiopsiezangen ist der Zangenkopf an seinem freien Längsende verjüngt ausgeführt, so dass er beim Hinausschieben in Körpergewebe eindringen kann. Bei herkömmlichen Biopsiezangen werden jedoch die bisher nur begrenzten Möglichkeiten zur Bewegung des Zangenkopfes mittels der Handhabungseinrichtung als Einschränkung empfunden. Des Weiteren bieten bekannte Punktionsbiopsiezangen nur eine als zu gering empfundene Punktionstiefe von maximal 2 cm.

Aus US 2012/0123201 A1 ist eine Biopsiezange gemäß dem Oberbegriff von Anspruch 1 bekannt. US 2010/0312141 A1 offenbart eine Biopsienadel sowie ein Verfahren zu deren Verwendung.

Aus JP 2002224121 A ist ein endoskopisches Werkzeug bekannt. JPH 10276967 A offenbart einen Katheter zur effizienten Verwendung mit verschiedenen Komponenten. Aus US 2009/0099414 A1 sind eine medizinische Behandlungsvorrichtung und ein Verfahren zur Diagnose oder endoskopischen Behandlung bekannt.

### Zusammenfassung der Erfindung

Vor diesem Hintergrund liegt der Erfindung die Aufgabe zugrunde, eine Biopsiezange, insbesondere eine Punktionsbiopsiezange, vorteilhaft weiterzubilden. Insbesondere wird angestrebt, dass mittels der Weiterbildung die aus dem Stand der Technik bekannten Einschränkungen möglichst weitgehend vermieden werden können. Insbesondere wird eine verbesserte Handhabbarkeit des Zangenkopfes angestrebt. Insbesondere wird auch eine im Vergleich zu dem Stand der Technik größere mögliche Punktionstiefe angestrebt.

Zur Lösung der gestellten Aufgabe schlägt die Erfindung gemäß Anspruch 1 vor, dass auf dem Führungskern mehrere in Betätigungsrichtung voneinander beabstandete Markierungen, insbesondere Zahlen, angebracht sind, wobei vorgesehen ist, dass die Hülse eine Ausnehmung aufweist, durch welche Markierungen ablesbar sind.

Der besagte Anschlag bildet bei der Anwendung der Biopsiezange einen Tiefenanschlag, durch dessen Gebrauch erreicht werden kann, dass der Vorschub des Zangenkopfes im Körpergewebe nur bis zu einer bestimmten wählbaren Tiefe erfolgt. Dies verbessert die Handhabung des Zangenkopfes. Der Tiefenanschlag wiederum erlaubt einen im Vergleich zum Stand der Technik größeren möglichen Verschiebeweg des Zangenkopfes relativ zu der flexiblen Ummantelung, um auch größere Punktionstiefen zu realisieren. Der Tiefenanschlag kann dabei verhindern, dass bei einer nur gewünschten geringeren als der maximalen Punktionstiefe versehentlich ein zu großer, mit der Gefahr von erheblichen Verletzungen verbundener Vorschub erfolgt. Wie noch nachfolgend beschrieben wird, kann eine erfindungsgemäße Biopsiezange vorzugsweise als Punktionsbiopsiezange ausgeführt sein, indem sich ihr Zangenkopf zu seinem distalen Längsende hin verjüngt. Eine erfindungsgemäße Punktionsbiopsiezange kann zum Beispiel zur Entnahme von Gewebe aus der Pankreaszystenwand und aus Lymphknoten Anwendung finden. Die Arbeitslänge bzw. Gesamtlänge einer erfindungsgemäßen Biopsiezange kann bis zu mehreren Metern (bspw. 5 bis 6 m) betragen.

Bei dem ersten Zug-/Schubteil handelt es sich um ein langgestrecktes Teil, das zu wahlweisen Übertragung von Zug- oder Schubkräften geeignet ist. Wenn die erste Betätigungseinrichtung in proximaler Richtung bewegt wird, kann mittels des ersten Zug-/Schubteils an dessen distalem Längsende eine Zugkraft übertragen werden. Wird hingegen die erste Betätigungseinrichtung in distaler Richtung bewegt, kann mittels des ersten Zug-/Schubteils an dessen distalem Längsende eine Schubkraft übertragen werden, mittels der sich der Zangenkopf in distaler Richtung bewegen lässt.

Es bestehen zahlreiche Möglichkeiten zur bevorzugten Weiterbildung einer erfindungsgemäßen Biopsiezange:
Es besteht die Möglichkeit, dass der Zangenkopf zwei Branchen aufweist, wobei eine Branche oder beide Branchen zwischen einer Geschlossenstellung und einer Offenstellung bewegbar ist oder sind. Vorzugsweise weist jede Branche eine löffelartige Ausnehmung auf. Anstelle von einer Hülse, die bei gelöster Klemmschraube auf dem Führungskern verschiebbar ist und die bei festgezogener Klemmschraube an dem Führungskern fixiert ist, könnte man insofern auch von einem Sicherheitsring sprechen. Bevorzugt ist, dass, wenn sich die erste Betätigungseinrichtung relativ zu der Halteeinrichtung in einer von einem proximalen Anschlag bestimmten proximalen Endposition befindet, der Zangenkopf in der flexiblen Ummantelung aufgenommen ist, und dass, wenn sich die Hülse und die erste Betätigungseinrichtung relativ zu der Halteeinrichtung in einer von einem distalen Anschlag der Halteeinrichtung bestimmten distalen Endposition befinden, der Zangenkopf und ein daran proximal angrenzender Längenteilabschnitt des ersten Zug-/Schubteils distal aus dem distalen Längsende der flexiblen Ummantelung hervorstehen. Zum Beispiel kann die distale Spitze des Zangenkopfes in der besagten Endposition um mindestens 5 cm, weiter vorzugsweise um zumindest 8 cm, aus der flexiblen Ummantelung hervorstehen.

Bei einem bevorzugten Ausführungsbeispiel ist vorgesehen, dass die Handhabungseinrichtung eine zweite Betätigungseinrichtung und ein Führungsteil, das mit der zweiten Betätigungseinrichtung eine zweite Längsführung bildet und das an der ersten Betätigungseinrichtung, insbesondere an ihrem proximalen Ende, befestigt oder ausgebildet ist, aufweist, dass die Biopsiezange ein zweites Zug-/Schubteil aufweist, das sich in seiner Längsrichtung verschieblich durch einen jeweiligen Hohlkanal der flexiblen Ummantelung, der Halteeinrichtung, der ersten Betätigungseinrichtung und des Führungsteils bis zu der zweiten Betätigungseinrichtung erstreckt, das an seinem proximalen Längsende an der zweiten Betätigungseinrichtung befestigt ist und das an seinem distalen Längsende mit den Branchen verbunden ist. Es kann sich um eine mittelbare oder um eine unmittelbare Verbindung handeln. Das zweite Zug-/Schubteil könnte man auch als Teil oder als Seele zur wahlweisen Zug- oder Schubübertragung bezeichnen. Wenn die zweite Betätigungseinrichtung in proximaler Richtung bewegt wird, kann mittels des zweiten Zug-/Schubteiles eine Zugkraft an dessen distalem Längsende übertragen werden. Wenn die zweite Betätigungseinrichtung entgegen in distaler Richtung bewegt wird, kann mittels des zweiten Zug-/Schubteils an dessen distalem Längsende eine in distaler Richtung wirkende Schubkraft ausgeübt werden.

Als zweckmäßig wird angesehen, dass das erste Zug-/Schubteil als biegsames Röhrchen, insbesondere als sog. Mikro-Röhrchen, ausgebildet ist oder zumindest ein Röhrchen umfasst, dass das zweite Zug-/Schubteil als Draht ausgebildet ist oder zumindest einen Draht umfasst und dass das zweite Zug-/Schubteil durch einen Hohlkanal des ersten Zug-/Schubteils in seiner Längsrichtung verschieblich hindurchgeführt ist. Das zweite Zug-/Schubteil kann bspw. auch mehrere, in Längsrichtung aneinandergefügte Drähte umfassen. Bevorzugt ist, dass der Draht in dem Röhrchen längsverschieblich geführt ist und dass das Röhrchen längsverschieblich in der flexiblen Ummantelung geführt ist.

Es besteht die Möglichkeit, dass eine Branche oder dass beide Branchen an dem Zangenkopf drehbar an einem Gelenk gehalten sind, dass sich an jede drehbare Branche proximal ein Hebelstück anschließt, das an seinem proximalen Ende mit je einem Hebelteil gelenkig verbunden ist und dass jedes Hebelteil an seinem proximalen Ende mit dem distalen Längsende des zweiten Zug-/Schubteils verbunden ist. Alternativ besteht die Möglichkeit, dass nur eine der beiden Branchen an dem Zangenkopf drehbar ist, und dass die andere Branche unbeweglich ist.

Bei einer zweckmäßigen Ausgestaltung ist vorgesehen, dass die erste Betätigungseinrichtung auf ihrer proximalen Seite mit dem als zweiter Führungsdorn ausgebildeten Führungsteil verbunden ist, dass die zweite Betätigungseinrichtung als Griffspule ausgebildet und in einer Betätigungsrichtung verschieblich auf den zweiten Führungsdorn aufgeschoben ist.

Eine erfindungsgemäße Biopsiezange kann als Punktionsbiopsiezange ausgebildet sein. Dazu besteht zum Beispiel die Möglichkeit, dass eine erste Branche länger als die zweite Branche ist, so dass sie sich in der Geschlossenstellung distal weiter als die zweite Branche erstreckt, wobei insbesondere vorgesehen ist, dass die erste Branche an ihrem freiem Längsende zu einer Spitze verjüngt ist. Insbesondere in diesem Zusammenhang wird als zweckmäßig angesehen, dass die erste Branche seitlich eine Ausnehmung aufweist, die geometrisch an eine teilweise oder vollständige Aufnahme der zweiten Branche in der Geschlossenstellung angepasst ist. Bevorzugt ist vorgesehen, dass eine oder beide Branchen schwenkbar an dem Zangenkopf gehalten und mittels des zweiten Zug-/Schubteils relativ zu dem distalen Längsende des ersten Zug-/Schubteils bewegbar ist oder sind. Dementsprechend kann der Zangenkopf entweder ein- oder beidseitig öffnend und schließend ausgestaltet sein.

Bevorzugt ist, dass die flexible Ummantelung aus Kunststoff, insbesondere aus Polyetheretherketon (PEEK) hergestellt ist. Dadurch kann im Vergleich zu herkömmlich bei endoskopischen Instrumenten bekannten metallischen Ummantelungen, die helixartig gewundene, aneinanderliegende Windungen besitzen, eine weniger steife Gestaltung erreicht werden. Zugleich kann eine für die Anforderung genügende Härte, Gleitfähigkeit und Verformbarkeit erzielt werden. Dabei wird unter einer flexiblen Ummantelung allgemein eine biegsame Ummantelung verstanden. Vorzugsweise sind auch das erste Zug-/Schubteil und das zweite Zug-/Schubteil jeweils biegsam bzw. flexibel ausgebildet.

Als zweckmäßig wird angesehen, dass die Handhabungseinrichtung an ihrem distalen Längsende einen Luer-Anschluss oder einen Luer-Lock-Anschluss zum Anschließen an einen Arbeitskanal eines Endoskops aufweist. Dies ermöglicht insbesondere einen abdichtenden Anschluss an einen Arbeitskanal.

### Kurze Beschreibung der Zeichnungen

Die Erfindung wird nachfolgend mit Bezug auf die beigefügten Figuren, welche bevorzugte Ausführungsbeispiele zeigen, weiter beschrieben. Im Einzelnen zeigt:
- Fig. 1: perspektivisch und mittels eines Aufbruches verkürzt eine erfindungsgemäße Biopsiezange gemäß einem ersten bevorzugten Ausführungsbeispiel, bei geöffnetem Zangenmaul;
- Fig. 2: eine Ausschnittsvergrößerung von Detail II aus Figur 1;
- Fig. 3: in einer ersten Seitenansicht die in Figur 1 gezeigte Biopsiezange, in einem ersten Gebrauchszustand,
- Fig. 3a: die in Figur 3 gezeigte Biopsiezange in dortiger Blickrichtung IIIa;
- Fig. 4: in einer zu Figur 3 vergleichbaren Seitenansicht die dort gezeigte Biopsiezange in einer weiteren Gebrauchsstellung;
- Fig. 5: in einer zu Figur 3 vergleichbaren Seitenansicht die dort gezeigte Biopsiezange, jedoch in einer weiteren Gebrauchsstellung,
- Fig. 5a: die in Figur 5 gezeigte Situation in dortiger Blickrichtung Va;
- Fig. 6: eine Ausschnittsvergrößerung von Detail VI gemäß Figur 5;
- Fig. 7: in einer zu Figur 5 vergleichbaren Seitenansicht die dortige Biopsiezange, jedoch in einer noch weiteren Gebrauchsstellung,
- Fig. 7a: die in Figur 7 gezeigte Situation in dortiger Blickrichtung VIIa;
- Fig. 8: eine Ausschnittsvergrößerung von Detail VIII gemäß Figur 7a;
- Fig. 9: in einer zu Figur 7 vergleichbaren Seitenansicht die dortige Biopsiezange, jedoch in einer noch weiteren Gebrauchsstellung,
- Fig. 9a: die in Figur 9 gezeigte Situation in dortiger Blickrichtung IXa;
- Fig. 10: eine Ausschnittsvergrößerung von Detail X gemäß Figur 9;
- Fig. 11: einen Längsschnitt entlang der Ebene XI-XI in Figur 9,
- Fig. 11a: eine Ausschnittsvergrößerung von Detail XIa in Figur 11,
- Fig. 11b: eine Ausschnittsvergrößerung von Detail XIb in Figur 11;
- Fig. 12: eine Schnittansicht entlang Schnittebene XII-XII in Figur 9a;
- Fig. 13: perspektivisch und mittels eines Aufbruches verkürzt eine erfindungsgemäße Biopsiezange gemäß einem zweiten bevorzugten Ausführungsbeispiel, bei geöffnetem Zangenmaul;
- Fig. 14: eine Ausschnittsvergrößerung von Detail XIV aus Figur 13;
- Fig. 15: in einer ersten Seitenansicht die in Figur 13 gezeigte Biopsiezange, in einem ersten Gebrauchszustand,
- Fig. 15a: die in Figur 15 gezeigte Biopsiezange in dortiger Blickrichtung XVa;
- Fig. 16: in einer zu Figur 15 vergleichbaren Seitenansicht die dort gezeigte Biopsiezange in einer weiteren Gebrauchsstellung;
- Fig. 17: in einer zu Figur 15 vergleichbaren Seitenansicht die dort gezeigte Biopsiezange, jedoch in einer weiteren Gebrauchsstellung,
- Fig. 17a: die in Figur 17 gezeigte Situation in dortiger Blickrichtung XVI-Ia;
- Fig. 18: eine Ausschnittsvergrößerung von Detail XVIII gemäß Figur 17;
- Fig. 19: in einer zu Figur 17 vergleichbaren Seitenansicht die dortige Biopsiezange, jedoch in einer noch weiteren Gebrauchsstellung,
- Fig. 19a: die in Figur 19 gezeigte Situation in dortiger Blickrichtung XI-Xa;
- Fig. 20: eine Ausschnittsvergrößerung von Detail XX gemäß Figur 19;
- Fig. 21: in einer zu Figur 19 vergleichbaren Seitenansicht die dortige Biopsiezange, jedoch in einer noch weiteren Gebrauchsstellung,
- Fig. 21a: die in Figur 21 gezeigte Situation in dortiger Blickrichtung XXIa;
- Fig. 22: eine Ausschnittsvergrößerung von Detail XXII gemäß Figur 21;
- Fig. 23: einen Längsschnitt entlang der Ebene XXIII-XXIII in Figur 21;
- Fig. 23a: eine Ausschnittsvergrößerung von Detail XXIIIa in Figur 23;
- Fig. 23b: eine Ausschnittsvergrößerung von Detail XXIIIb in Figur 23;
- Fig. 24: eine Schnittansicht entlang Schnittebene XXIV-XXIV in Figur 21a;
- Fig. 25: perspektivisch und mittels eines Aufbruches verkürzt eine erfindungsgemäße Biopsiezange gemäß einem dritten bevorzugten Ausführungsbeispiel, bei geöffnetem Zangenmaul; und
- Fig. 26: einen Teilschnitt entlang Schnittebene XXIV-XXIV in Figur 25.

### Beschreibung der Ausführungsformen

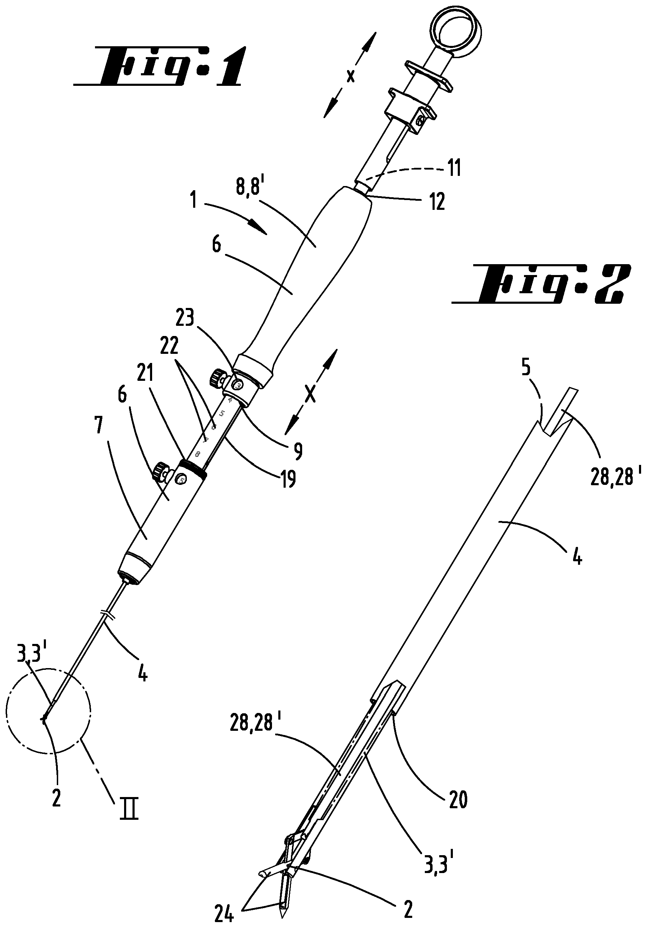

Mit Bezug auf die Figuren 1 bis 12 wird zunächst ein erstes Ausführungsbeispiel einer erfindungsgemäßen Biopsiezange 1 vorgestellt. In dem Beispiel ist die Biopsiezange 1 als Punktionsbiopsiezange ausgebildet. Sie umfasst einen Zangenkopf 2 sowie ein in dem Beispiel als dünne Metallhülse (sog. "Mikroröhrchen") ausgebildetes erstes Zug-/Schubteil 3, an dessen distalem Längsende der Zangenkopf 2 befestigt ist. Gemäß dem üblichen Sprachgebrauch bedeutet die Bezeichnung distal eine von einem Benutzer bzw. Operateur der Biopsiezange 1 abgewandte Position oder Richtung, während der Begriff proximal eine dem Benutzer bzw. Operateur zugewandte Lage oder Richtung bezeichnet. Die Biopsiezange 1 umfasst eine biegbare Ummantelung 4, die in ihrem Inneren einen sich entlang ihrer gesamten Länge erstreckenden Hohlkanal aufweist. Durch diesen ist das erste Zug-/Schubteil 3 in seiner Längsrichtung relativ zu der flexiblen Ummantelung 4 längsverschieblich hindurchgeführt. Die Biopsiezange 1 umfasst eine Handhabungseinrichtung 6, die ihrerseits eine Halteeinrichtung 7 und eine erste Betätigungseinrichtung 8 aufweist. Die Halteeinrichtung 7 kann von dem Operateur mit einer Hand festgehalten werden, während die erste Betätigungseinrichtung 8 von der zweiten Hand ergriffen und relativ zu der Halteeinrichtung 7 in einer mit X bezeichneten Betätigungsrichtung bewegt werden kann. Dazu bilden die Halteeinrichtung 7 und die erste Betätigungseinrichtung 8 gemeinsam eine Längsführung 9, mittels der die erste Betätigungseinrichtung 8 an der Halteeinrichtung 7 relativ zu ihr entlang der Betätigungsrichtung X verschiebbar geführt ist.

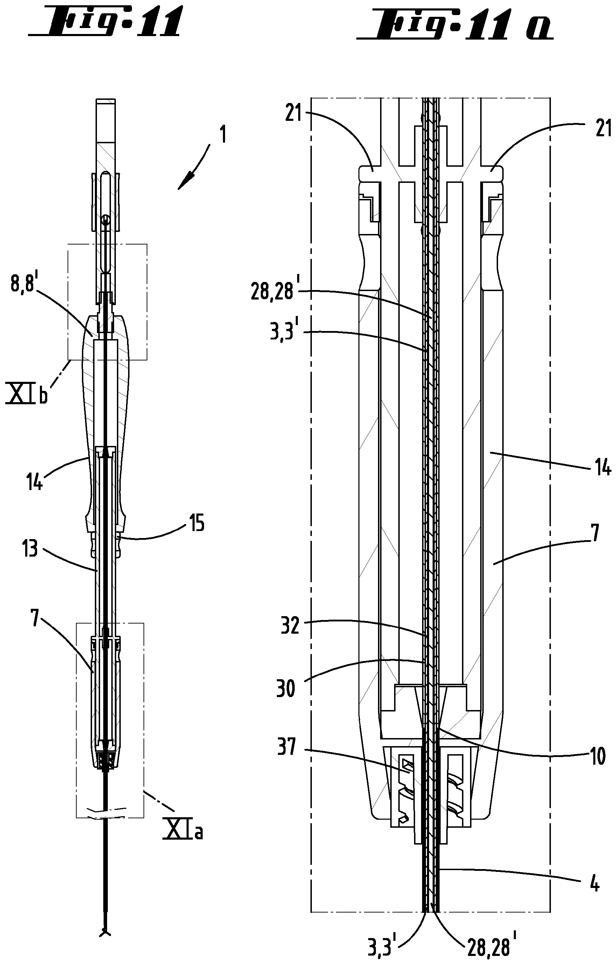

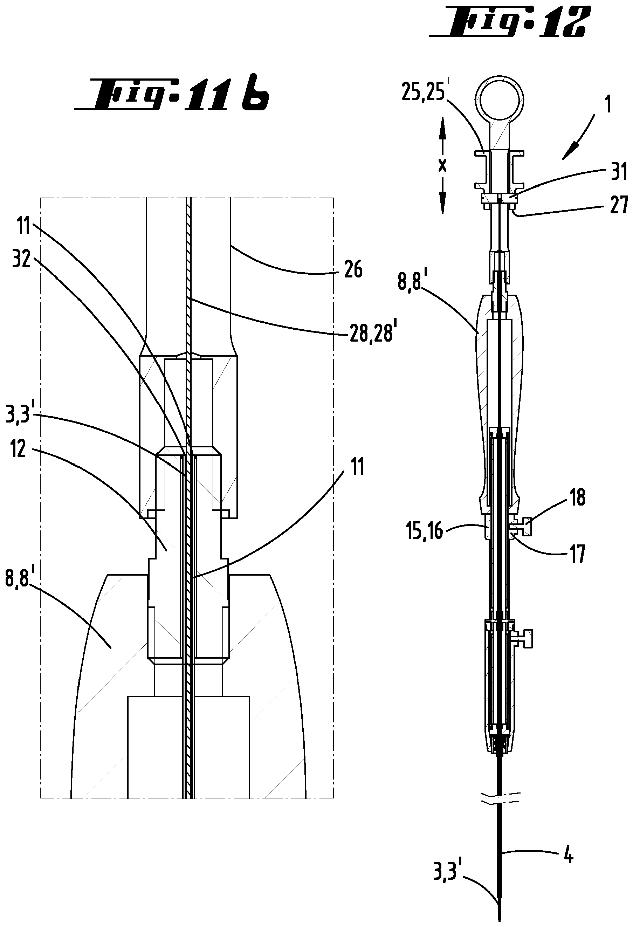

Die flexible Ummantelung 4 ist in dem Beispiel an ihrem proximalen Längsende 10 mit der Halteeinrichtung 7 fest verbunden. Das erste Zug-/Schubteil 3 ist an seinem proximalen Längsende 11 (vgl. Figur 11b) mit der ersten Betätigungseinrichtung 8 fest verbunden. In dem Beispiel umfasst die erste Betätigungseinrichtung 8 einen Handgriff 8', in dessen proximales Längsende eine Befestigungshülse 12 zur darin inneren Befestigung des ersten Zug-/Schubteils 3 eingeschraubt ist. In dem Beispiel weist die Halteeinrichtung 7 einen Führungskern 13 auf. Die erste Betätigungseinrichtung 8 umfasst eine integral in dem Handgriff 8' ausgebildete Führungshülse 14. Diese ist zur gemeinsamen Bildung der Längsführung 9 auf den Führungskern 13 aufgeschoben und darauf entlang der Betätigungsrichtung X verschiebbar. Dabei ist ein Außendurchmesser des Führungskerns 13 an einen Innendurchmesser der Führungshülse 14 so angepasst, dass sich eine spielarme Längsführung ergibt.

Die Biopsiezange 1 weist eine Wegbegrenzungseinrichtung 15 auf, bei der es sich in dem Beispiel um eine Hülse 16 handelt. Diese ist auf den Führungskern 13 aufgeschoben. Der Innendurchmesser der Hülse 16 entspricht im Wesentlichen dem Außendurchmesser des Führungskerns 13, so dass eine spielarme Führung entlang der Betätigungsrichtung X resultiert. Die Hülse 16 besitzt in ihrer Wandung eine durchgehende radiale Gewindebohrung 17, durch welche eine Klemmschraube 18 geschraubt ist. Wird die Klemmschraube eingedreht, drückt sie schließlich gegen den Führungskern 13, wodurch die Wegbegrenzungseinrichtung 15 an der Längsführung 9 in einer gewünschten Position festgeklemmt wird. In diesem befestigten Zustand bildet die Wegbegrenzungseinrichtung 15 einen Anschlag, der den möglichen Verschiebeweg der ersten Betätigungseinrichtung 8 relativ zu der Halteeinrichtung 7 in distaler Richtung entlang der Betätigungsrichtung X begrenzt.

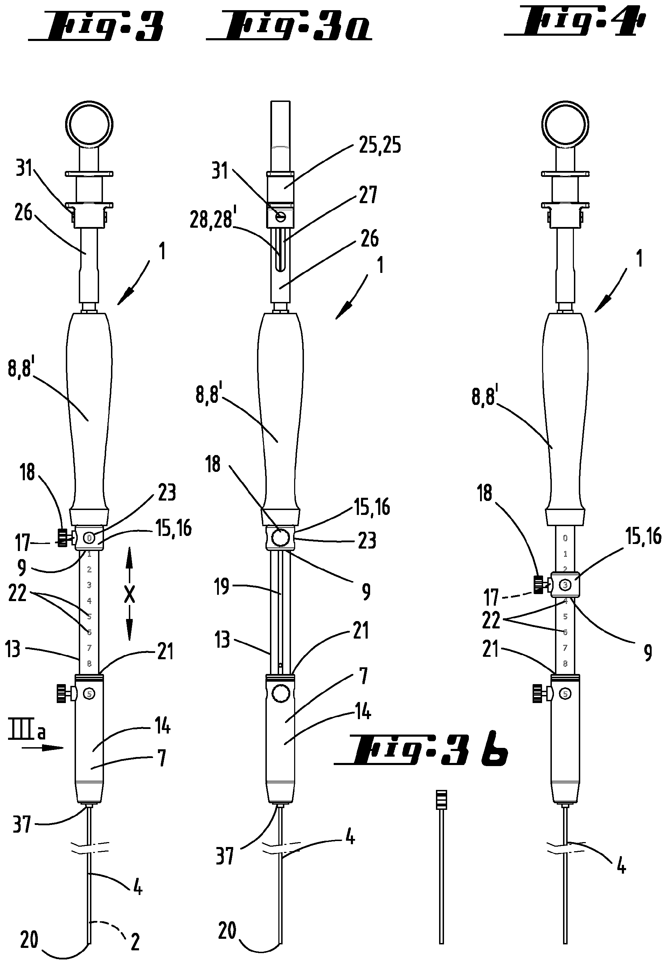

In den Figuren 3, 3a ist ein Gebrauchszustand gezeigt, in welchem sich die erste Betätigungseinrichtung 8 relativ zu der Halteeinrichtung 7 in einer von einem proximalen Anschlag bestimmten proximalen Endposition befindet. Zu diesem Zweck ist in dem Beispiel in dem Führungskern 13 eine Längsnut 19 ausgebildet, die an ihrem proximalen Längsende geschlossen ist; an dem distalen Längsende der Führungshülse 14 greift ein (in den Figuren nicht mit dargestellter) nach radial innen weisender Vorsprung in die Längsnut 19 ein. Beim Zurückziehen der Betätigungseinrichtung 8 (also in proximaler Richtung) relativ zu der Halteeinrichtung 7 tritt der besagte Vorsprung in proximaler Richtung gegen die Stirnwand der Längsnut 19, wodurch der proximale Anschlag gebildet wird. In diesem Zustand befindet sich der Zangenkopf 2 vollständig im Inneren der flexiblen Ummantelung 4. In diesem Zustand kann auf die distale Spitze 20 der Ummantelung 4 bspw. eine Kappe oder dergleichen als Schutz bzw. als Transportsicherung aufgesteckt werden. Eine solche Kappe kann bspw. aus einem kurzen Längenabschnitt eines Schlauches (vorzugsweise entsprechend der flexiblen Ummantelung 4) und einem auf sein eines Längsende aufgeschobenen Steckadapter gebildet sein und ist exemplarisch in Figur 3b gezeigt. Die Wegbegrenzungseinrichtung 15 ist bei dem in den Figuren 3, 3a bezeichneten Gebrauchszustand, in welchem sich die Betätigungseinrichtung 8 an ihrer proximalen Endposition befindet, weitestmöglich, d.h. bis zum Anschlag gegen die Betätigungseinrichtung 8, verschoben und in dieser Position festgeklemmt. Dies verhindert, bspw. während eines Transports, einen ungewollten Austritt des Zangenkopfes 2 aus der Ummantelung 4.

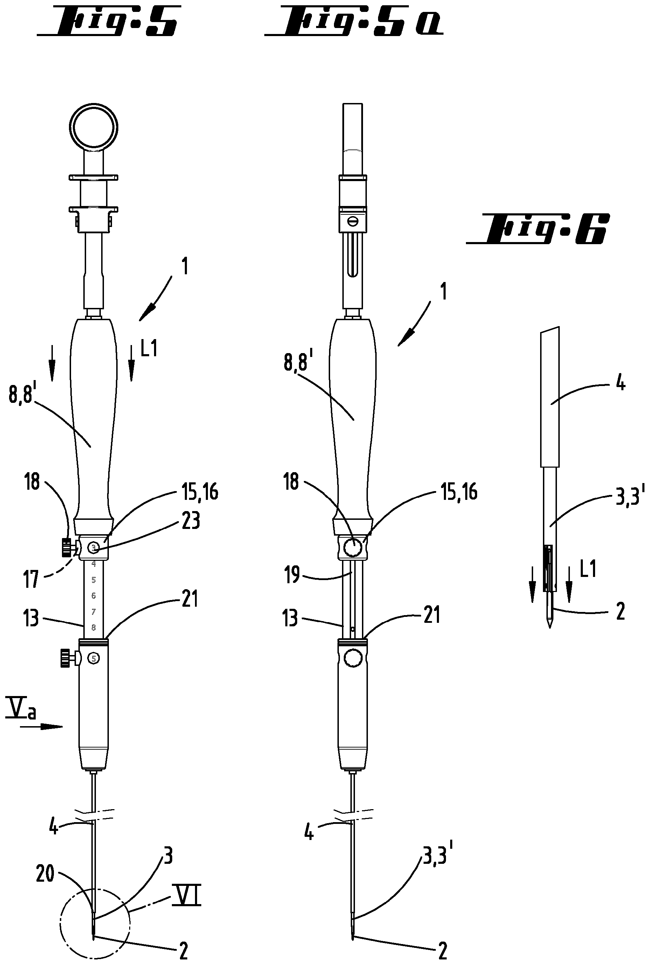

Abweichend davon wurde bei dem in Figur 4 gezeigten Gebrauchszustand die Hülse 16 bei gelöster Klemmschraube 18 einen Teil der möglichen Wegstrecke entlang der Betätigungsrichtung X verschoben und dort geklemmt. Die Figuren 5, 5a zeigen einen Gebrauchszustand, in welchem die Betätigungseinrichtung 8 relativ zu der Halteeinrichtung 7 entlang der Betätigungsrichtung X in distaler Richtung bis zum Anschlag gegen die Hülse 16 vorgeschoben wurde. Da die Betätigungseinrichtung 8 fest mit dem proximalen Längsende des ersten Zug-/Schubteils 3 verbunden ist, wird dieses durch die beschriebene Verlagerung ebenfalls in distaler Richtung bewegt, so dass schließlich der Zangenkopf 2 und ein sich hieran proximal anschließender Längenabschnitt des ersten Zug-/Schubteils 3 aus der flexiblen Ummantelung 4 distal heraustritt. In den Figuren 5, 5a ist dieser exemplarisch gewählte Betätigungsweg mit L1 bezeichnet. Die Länge bzw. die Wegstrecke, um die der Zangenkopf 2 dabei maximal über die distale Spitze 20 der Ummantelung 4 hervorstehen kann, wird mittels der Wegbegrenzungseinrichtung 15 als Tiefenanschlag begrenzt. Anhand der Figuren wird deutlich, dass sich die Wegbegrenzungseinrichtung 15 bei Bedarf zur Vergrößerung dieser maximalen Wegstrecke entlang des Führungskerns 13 in distaler Richtung verschieben ließe, bis sie gegen einen Vorsprung 21 der Halteeinrichtung 7 tritt. Dies ermöglicht für die Betätigungseinrichtung 8 den maximalen Verschiebeweg. In dem gewählten Beispiel beträgt dieser 8 cm. Wird die Betätigungseinrichtung 8 dabei in distaler Richtung bis gegen die Hülse 16 verschoben, befindet sie sich in ihrer distalen Endposition; dabei tritt der Zangenkopf 2 und ein sich daran proximal anschließender Teilabschnitt des ersten Zug-/Schubteils 3 um mehrere Zentimeter distal aus dem distalen Längsende der flexiblen Ummantelung 4 heraus. In dem Beispiel sind auf dem Führungskern 13 acht in Betätigungsrichtung X voneinander beabstandete Markierungen 22 angebracht. In dem Beispiel handelt es sich um Ziffern, welche den Verschiebeweg in Zentimetern bezeichnen. In der Hülse 16 ist eine Ausnehmung 23 ausgebildet, wodurch die Markierungen 22 ablesbar sind.

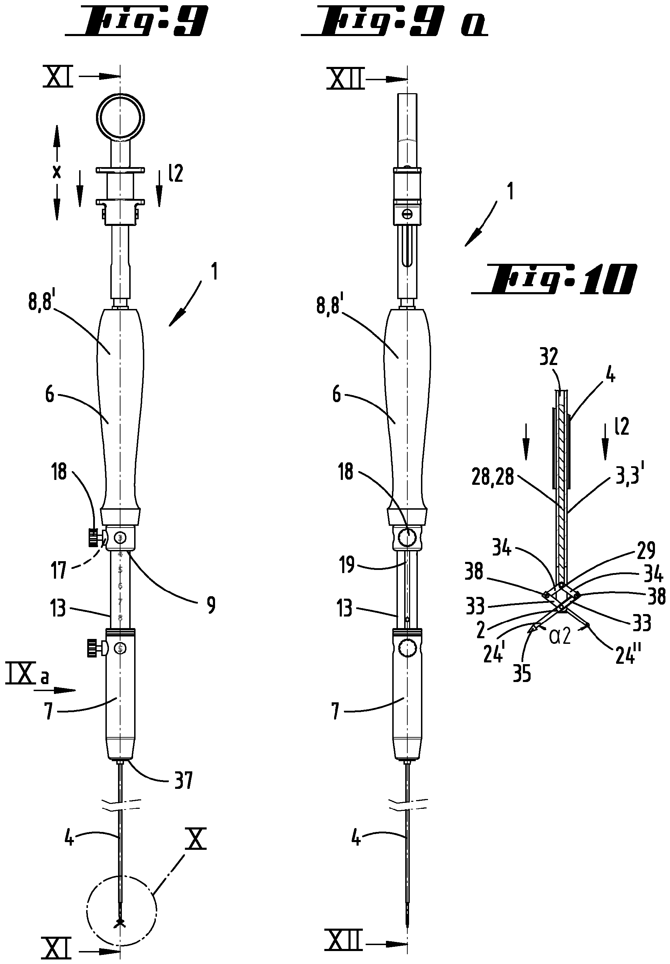

Bei dem gewählten Ausführungsbeispiel umfasst der Zangenkopf 2 zwei Branchen 24. Anstelle von Branchen könnte auch von vorderen Zangenschenkeln gesprochen werden. Beide Branchen 24 sind zwischen einer Geschlossenstellung und einer maximalen Offenstellung des Zangenkopfes bewegbar. Zur Bewegung ist vorgesehen, dass die Handhabungseinrichtung 6 eine zweite Betätigungseinrichtung 25 sowie ein ihr zugeordnetes Führungsteil 26, das mit der zweiten Betätigungseinrichtung 25 eine zweite Längsführung 27 bildet, aufweist. Das Führungsteil 26 ist an dem proximalen Längsende des Handgriffes 8' befestigt und erstreckt sich in dessen geradliniger Verlängerung. Zur Bewegung der Branchen umfasst die Biopsiezange 1 des Weiteren ein zweites Zug-/Schubteil 28. In dem Beispiel handelt es sich um einen Draht 28', der aus mehreren, an ihren Längsenden miteinander verbundenen Drahtstücken gebildet ist. Der Draht 28' steht an seinem distalen Längsende 29 (vgl. Figur 10) in Verbindung mit den Branchen 24. Von dort erstreckt sich der Draht 28' in proximaler Richtung durch einen Hohlkanal im Inneren des als Röhrchen 3' ausgebildeten ersten Zug-/Schubteils 3. Dabei erstreckt sich der Draht 28' in dem Röhrchen 3' in einem Längenteilabschnitt zugleich innerhalb der Ummantelung 4. An dem proximalen Längsende der Ummantelung 4 schließt ein Stützröhrchen 30 an, das sich bis zu dem proximalen Längsende des Führungskerns 13 erstreckt. Auch durch das Stützröhrchen 30 verläuft das Röhrchen 3' und darin der Draht 28'. In proximaler Richtung über das Stützröhrchen 30 hinaus erstreckt sich der Draht 28' weiter durch das erste Zug-/Schubteil 3 bzw. durch das Röhrchen 3', dessen proximales Längsende im Inneren der Befestigungshülse 12 befestigt ist. Der Draht 28' erstreckt sich von dort weiter in proximaler Richtung bis zu der zweiten Betätigungseinrichtung 25. Dabei handelt es sich in dem Beispiel um eine Griffspule 25', die entlang des Führungsteils 26 in einer Betätigungsrichtung x verschiebbar ist. Die Betätigungseinrichtung 25 weist einen Haltebolzen 31 auf, an dem das proximale Längsende des zweiten Zug-/Schubteils 28, d.h. des Drahtes 28' befestigt ist. Der im Inneren des Röhrchens 3' in dessen Längsrichtung verlaufende Hohlkanal, durch den sich der Draht 28' erstreckt, ist in den Figuren mit dem Bezugszeichen 32 bezeichnet (vgl. Figur 10).

Beide Branchen 24 sind an dem Zangenkopf 2 drehbar an einem Gelenk 38 gehalten. An jede Branche 24 schließt sich proximal ein Hebelstück (jeweils mit 33 bezeichnet) an, das an seinem proximalen Ende mit je einem Hebelteil 34 gelenkig verbunden ist. Jedes Hebelteil 34 ist wiederum an seinem proximalen Ende mit dem distalen Längsende 29 des zweiten Zug-/Schubteils 28, d.h. des Drahtes 28', verbunden.

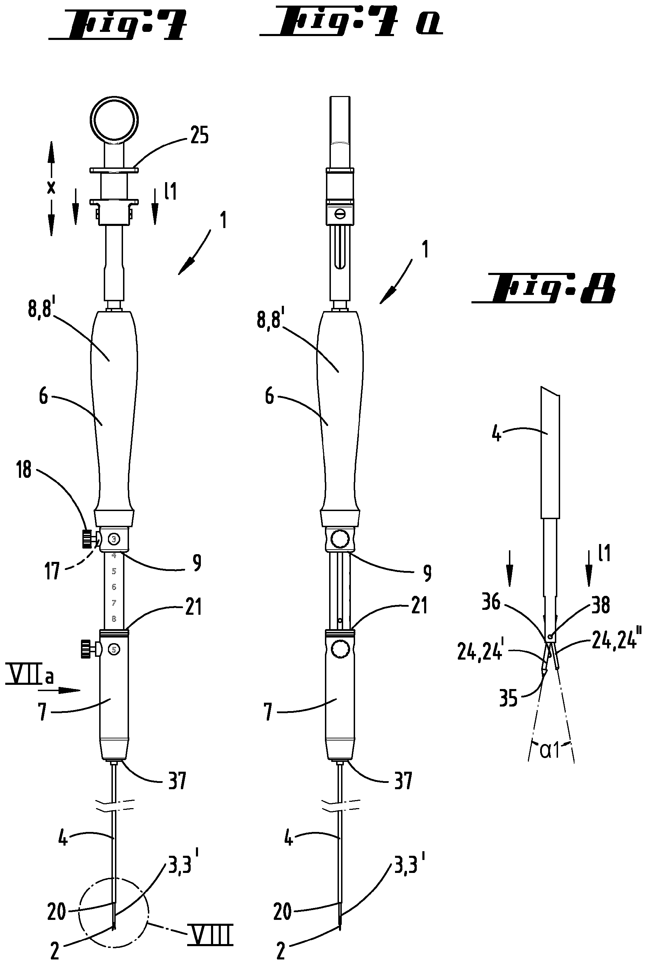

Das Führungsteil 26 ist als zweiter Führungsdorn ausgebildet, und die Griffspule 25' ist in der Betätigungsrichtung x verschieblich auf dem zweiten Führungsdorn aufgeschoben. Die Betätigungsrichtung x verläuft parallel zu der schon beschriebenen Betätigungsrichtung X der ersten Betätigungseinrichtung 8. In den Figuren 5, 5a befindet sich die zweite Betätigungseinrichtung 25 relativ zu dem Führungsteil 26 in ihrer proximalen Endposition. Dies gilt demzufolge auch für das distale Längsende 29 des Drahtes 28'. Dies bewirkt eine Streckung der Hebelstücke 33 und Hebelteile 34, so dass die Branchen 24 bzw. das Zangenmaul geschlossen sind (vgl. Figur 6). Im Vergleich dazu zeigen die Figuren 7, 7a einen Gebrauchszustand, in welchem die zweite Betätigungseinrichtung 25 um eine Wegstrecke 11 in distaler Richtung verlagert wurde. Dies bewirkt eine Öffnung der Branchen 24 um einen Öffnungswinkel α1. Die Figuren 9, 9a zeigen im Vergleich dazu einen Gebrauchszustand, in welchem die zweite Betätigungseinrichtung 25 um eine vergleichsweise größere Wegstrecke 12 in distaler Richtung relativ zu dem Führungsteil 26 verschoben wurde. Dies bewirkt (vgl. Figur 10) eine weitestmögliche Öffnung der Branchen 24, die nun einen Öffnungswinkel a2 einschließen.

Figur 10 zeigt auch, dass eine erste Branche 24' länger als eine zweite Branche 24" ausgebildet ist. Die längere Branche 24' ist an ihrem freien Längsende zu einer Spitze 35 verjüngt. Dadurch ist die Biopsiezange 1 als Punktionsbiopsiezange ausgebildet. Die erste Branche 24' weist seitlich eine Ausnehmung 36 auf, die geometrisch zur Aufnahme der zweiten Branche 24" in der Geschlossenstellung angepasst ist.

In dem Beispiel ist die flexible Ummantelung 4 aus dem Kunststoff Polyetheretherketon (PEEK) hergestellt. Die Handhabungseinrichtung 6 weist an ihrem distalen Längsende an der Halteeinrichtung 7 einen Luer-Lock-Anschluss 37 zum Anschließen an einen Arbeitsgang eines Endoskops auf. Bei dem in den Figuren 1 bis 12 gezeigten ersten Ausführungsbeispiel der Biopsiezange 1 ist der Luer-Lock-Anschluss 37 in eine Hülse 39 eingesetzt, die auf eine distale Verlängerung des Führungskerns 13 aufgeschoben und daran mittels einer Klemmschraube gehalten ist.

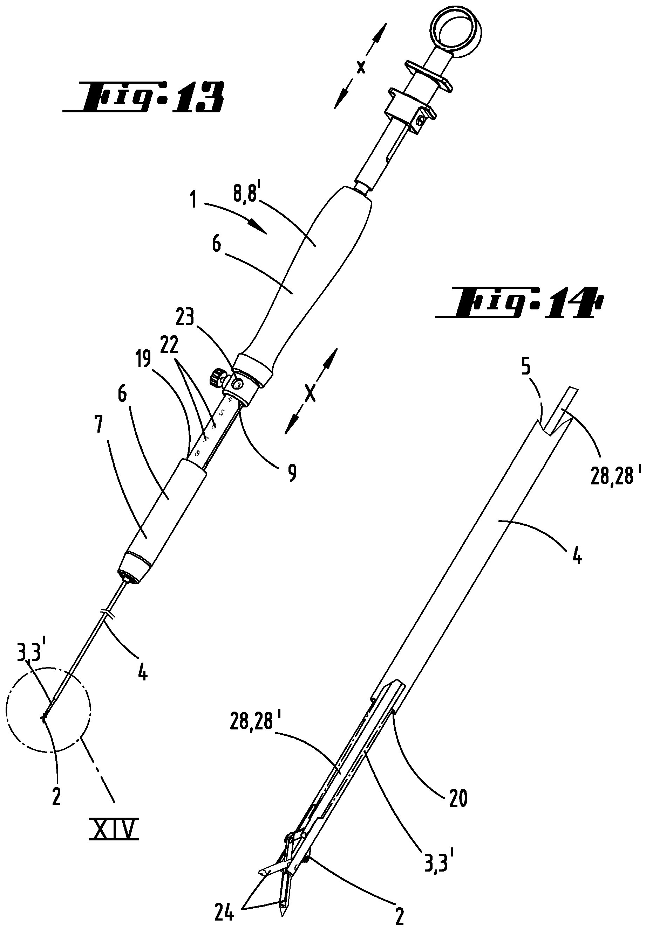

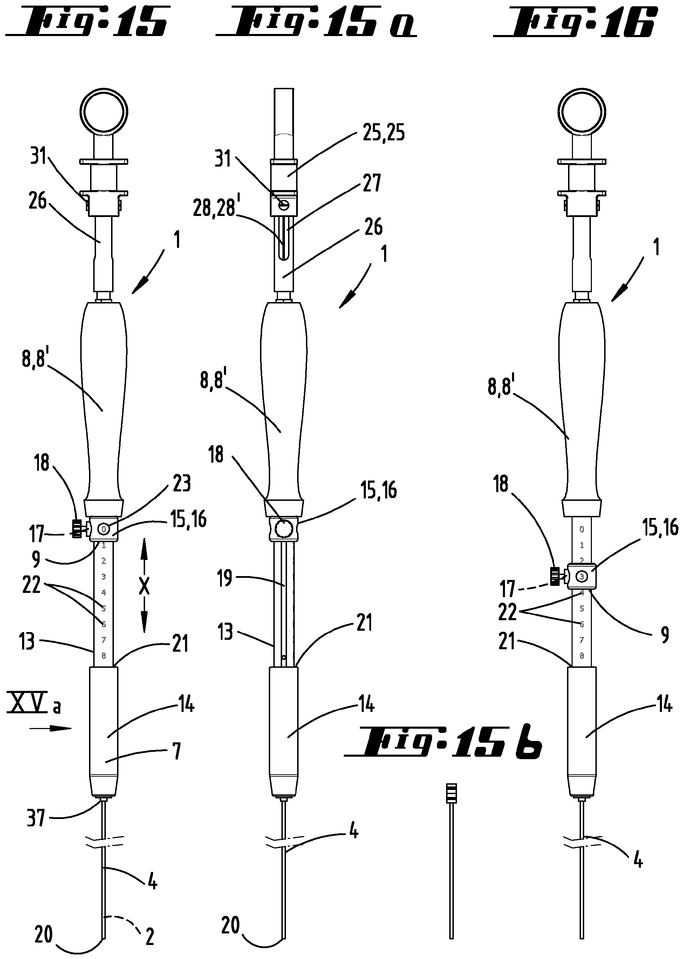

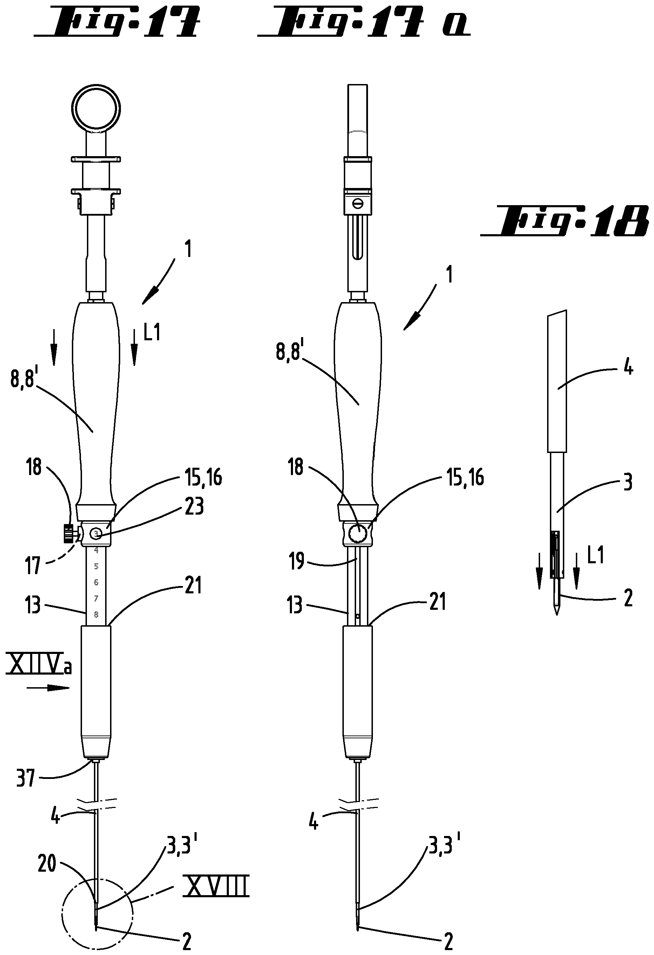

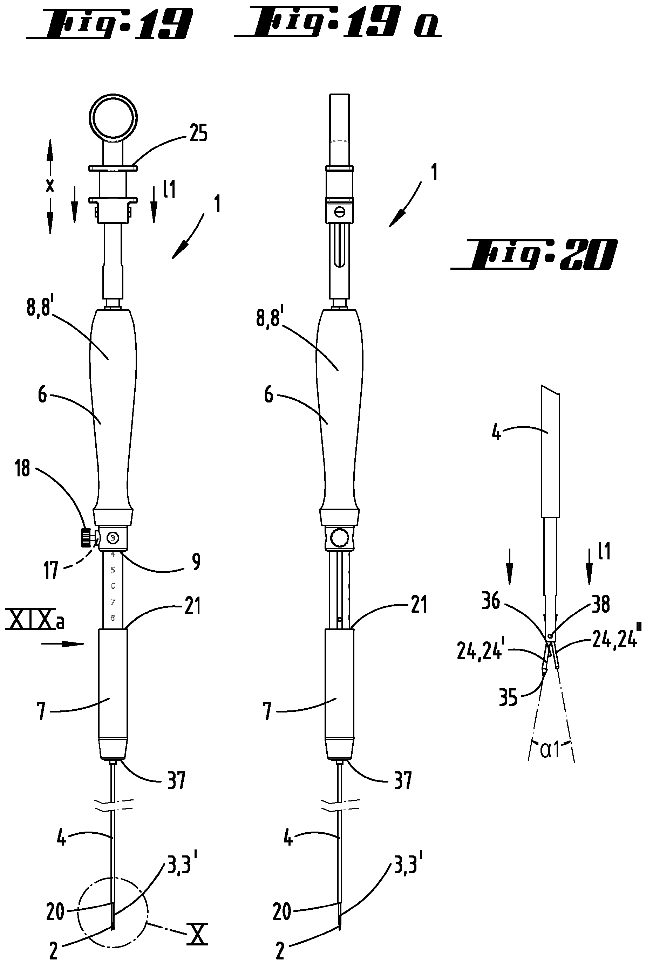

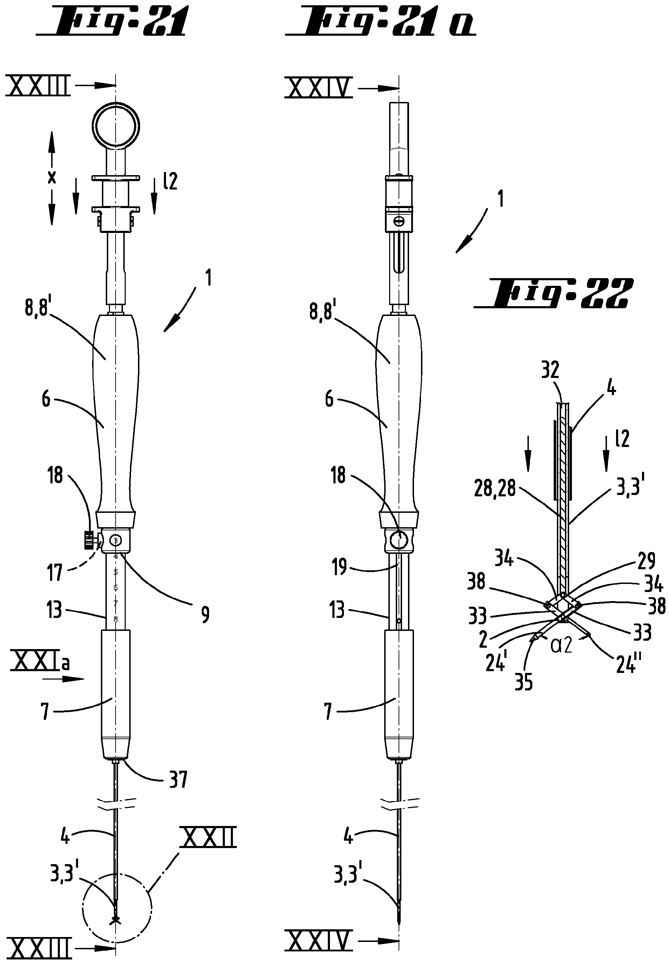

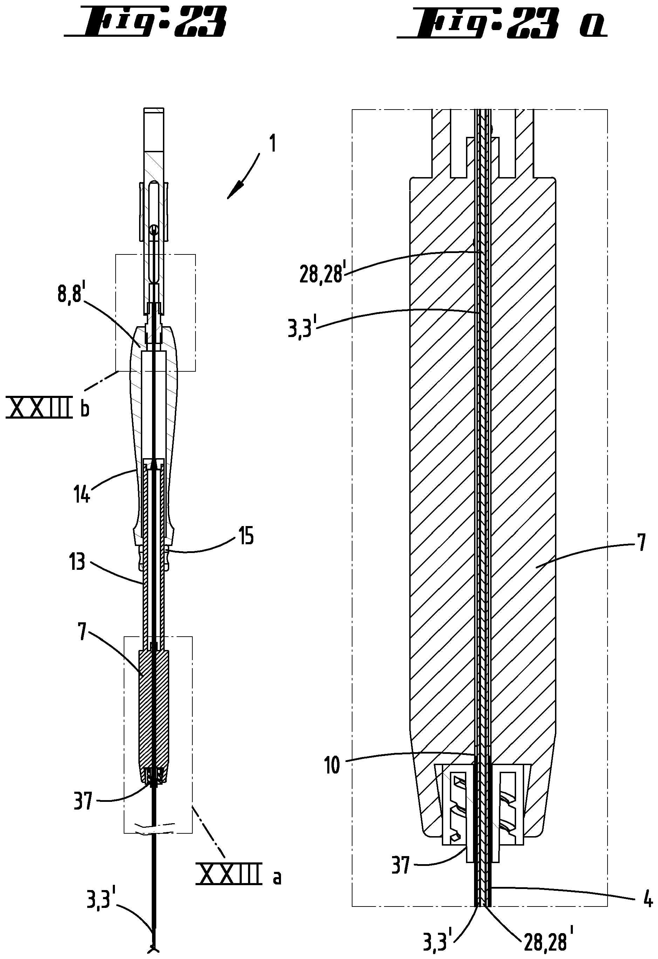

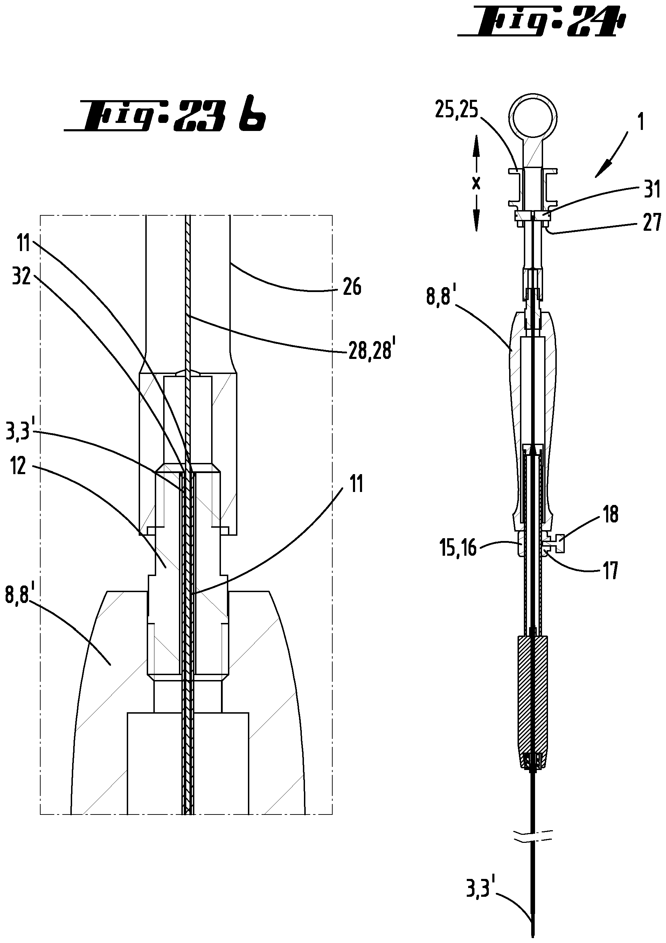

Abweichend davon ist bei dem in den Figuren 13 bis 24 gezeigten zweiten Ausführungsbeispiel der Biopsiezange 1 der Luer-Lock-Anschluss 37 unmittelbar in eine einstückig an den Führungskern 13 angeformte distale Verlängerung eingesetzt.

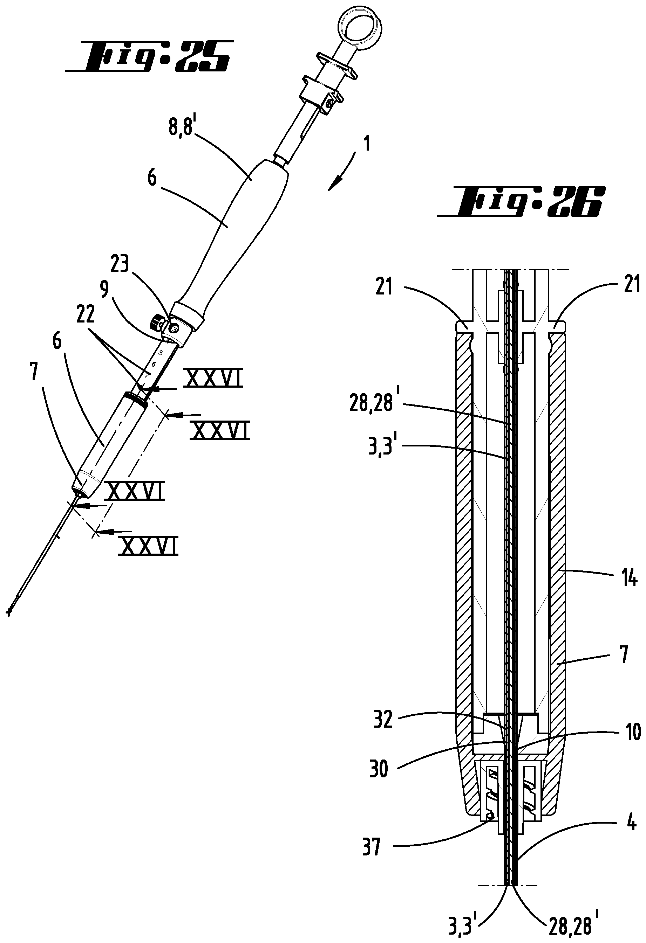

Das in den Figuren 25 und 26 gezeigte dritte Ausführungsbeispiel unterscheidet sich nur geringfügig von dem ersten, in den Figuren 1 bis 12 gezeigten Beispiel. Ein Unterschied liegt darin, dass dort die den Luer-Lock-Anschluss 37 aufnehmende Hülse nicht längsverschieblich an der Verlängerung des Führungskerns 13 gehalten ist, sondern in einer proximalen Endposition daran festgeklipst ist.

## Patentansprüche

1. Biopsiezange (1), insbesondere Punktionsbiopsiezange,
wobei die Biopsiezange (1) einen Zangenkopf (2) umfasst,
wobei die Biopsiezange (1) ein erstes Zug-/Schubteil (3) umfasst, an dessen distalem Längsende der Zangenkopf (2) angebracht ist,
wobei die Biopsiezange (1) eine flexible Ummantelung (4) umfasst, die einen Hohlkanal (5) aufweist, durch den das erste Zug-/Schubteil (3) relativ zu der flexiblen Ummantelung (4) längsverschieblich hindurchgeführt ist,
wobei die Biopsiezange (1) eine Handhabungseinrichtung (6) umfasst, die eine Halteeinrichtung (7) und eine erste Betätigungseinrichtung (8) aufweist,
wobei die Halteeinrichtung (7) und die erste Betätigungseinrichtung (8) gemeinsam eine erste Längsführung (9) bilden, mittels der die erste Betätigungseinrichtung (8) an der Halteeinrichtung (7) relativ zu ihr entlang einer Betätigungsrichtung (X) verschiebbar geführt ist,
wobei die flexible Ummantelung (4), insbesondere an ihrem proximalen Längsende (10), mit der Halteeinrichtung (7) verbunden ist,
wobei das erste Zug-/Schubteil (3), insbesondere an seinem proximalen Längsende (11), mit der ersten Betätigungseinrichtung (8) verbunden ist, wobei die Biopsiezange (1) eine Wegbegrenzungseinrichtung (15) umfasst, die an der ersten Längsführung (9) an in Bezug auf die genannte Betätigungsrichtung (X) wählbaren Positionen lösbar befestigbar ist und die in befestigtem Zustand einen Anschlag bildet, der einen Verschiebeweg der ersten Betätigungseinrichtung (8) relativ zu der Halteeinrichtung (7) in distaler Richtung begrenzt, wobei die Halteeinrichtung (7) einen Führungskern aufweist, wobei die erste Betätigungsrichtung (8) eine Führungshülse (14) aufweist, wobei der Führungskern (13) und die Führungshülse gemeinsam die erste Längsführung (9) ausbilden, wobei die Wegbegrenzungseinrichtung (15) eine Hülse (16) aufweist, die auf den Führungskern (13) aufgeschoben ist, wobei die Hülse (16) in ihrer Wandung eine Gewindebohrung (17) aufweist, in die eine Klemmschraube (18) eingeschraubt ist, wobei die Hülse (16) bei gelöster Klemmschraube (18) auf dem Führungskern (13) verschiebbar ist und wobei die Hülse (16) bei festgezogener Klemmschraube (18) an dem Führungskern (13) fixiert ist, **dadurch gekennzeichnet, dass** auf dem Führungskern (13) mehrere in Betätigungsrichtung (X) voneinander beabstandete Markierungen (22), insbesondere Zahlen, angebracht sind, wobei vorgesehen ist, dass die Hülse eine Ausnehmung (23) aufweist, durch welche Markierungen (22) ablesbar sind.

2. Biopsiezange (1) gemäß Anspruch 1, **dadurch gekennzeichnet, dass** der Zangenkopf (2) zwei Branchen aufweist, wobei eine Branche oder beide Branchen zwischen einer Geschlossenstellung und einer Offenstellung bewegbar ist oder sind.

3. Biopsiezange (1) gemäß einem oder mehreren der vorangehenden Ansprüche, **dadurch gekennzeichnet, dass**, wenn sich die erste Betätigungseinrichtung (8) relativ zu der Halteeinrichtung (7) in einer von einem proximalen Anschlag bestimmten proximalen Endposition befindet, der Zangenkopf (2) in der flexiblen Ummantelung (4) aufgenommen ist, und dass, wenn sich die Hülse (16) und die erste Betätigungseinrichtung (8) relativ zu der Halteeinrichtung (7) in einer von einem distalen Anschlag der Halteeinrichtung (7) bestimmten distalen Endposition befinden, der Zangenkopf (2) und ein daran proximal angrenzender Längenteilabschnitt des ersten Zug-/Schubteils (3) distal aus dem distalen Längsende der flexiblen Ummantelung (4) hervorstehen.

4. Biopsiezange (1) gemäß einem oder mehreren der vorangehenden Ansprüche, **dadurch gekennzeichnet, dass** die Handhabungseinrichtung (6) eine zweite Betätigungseinrichtung (25) und ein Führungsteil (26), das mit der zweiten Betätigungseinrichtung (25) eine zweite Längsführung (27) bildet und das an der ersten Betätigungseinrichtung (8), insbesondere an ihrem proximalen Ende, befestigt oder ausgebildet ist, aufweist, dass die Biopsiezange (1) ein zweites Zug-/Schubteil (28) aufweist, das sich in seiner Längsrichtung verschieblich durch einen jeweiligen Hohlkanal der flexiblen Ummantelung (4), der Halteeinrichtung (7), der ersten Betätigungseinrichtung (8) und des Führungsteils (26) bis zu der zweiten Betätigungseinrichtung (25) erstreckt, das an seinem proximalen Längsende an der zweiten Betätigungseinrichtung (25) befestigt ist und das an seinem distalen Längsende (29) mit den Branchen (24) verbunden ist.

5. Biopsiezange (1) gemäß einem oder mehreren der vorangehenden Ansprüche, **dadurch gekennzeichnet, dass** das erste Zug-/Schubteil (3) als Röhrchen (3') ausgebildet ist oder zumindest ein Röhrchen (3') umfasst, dass das zweite Zug-/Schubteil (28) als Draht (28') ausgebildet ist oder zumindest einen Draht (28') umfasst und dass das zweite Zug-/Schubteil (28) durch einen Hohlkanal (30) des ersten Zug-/Schubteils (3) in seiner Längsrichtung verschieblich hindurchgeführt ist.

6. Biopsiezange gemäß einem oder mehreren der vorangehenden Ansprüche, **dadurch gekennzeichnet, dass** eine Branche oder dass beide Branchen (24) an dem Zangenkopf (2) drehbar an einem Gelenk (38) gehalten sind, dass sich an jede drehbare Branche (24) proximal ein Hebelstück (33) anschließt, das an seinem proximalen Ende mit je einem Hebelteil (34) gelenkig verbunden ist und dass jedes Hebelteil (34) an seinem proximalen Ende mit dem distalen Längsende (29) des zweiten Zug-/Schubteils (28) verbunden ist.

7. Biopsiezange (1) gemäß einem oder mehreren der vorangehenden Ansprüche, **dadurch gekennzeichnet, dass** die erste Betätigungseinrichtung (8) auf ihrer proximalen Seite mit dem als zweiter Führungsdorn ausgebildeten Führungsteil (26) verbunden ist, dass die zweite Betätigungseinrichtung (25) als Griffspule (25') ausgebildet und in einer Betätigungsrichtung (x) verschieblich auf den zweiten Führungsdorn aufgeschoben ist.

8. Biopsiezange (1) gemäß einem oder mehreren der vorangehenden Ansprüche, **dadurch gekennzeichnet, dass** eine erste Branche (24') länger als die zweite Branche (24") ist, so dass sie sich in der Geschlossenstellung distal weiter als die zweite Branche (24") erstreckt, wobei insbesondere vorgesehen ist, dass die erste Branche (24') an ihrem freiem Längsende zu einer Spitze (35) verjüngt ist.

9. Biopsiezange (1) gemäß einem oder mehreren der vorangehenden Ansprüche, **dadurch gekennzeichnet, dass** die erste Branche (24') seitlich eine Ausnehmung (36) aufweist, die geometrisch an eine teilweise oder vollständige Aufnahme der zweiten Branche (24") in der Geschlossenstellung angepasst ist.

10. Biopsiezange (1) gemäß einem oder mehreren der vorangehenden Ansprüche, **dadurch gekennzeichnet, dass** eine oder beide Branchen (24) schwenkbar an dem Zangenkopf (2) gehalten und mittels des zweiten Zug-/Schubteils (28) relativ zu dem distalen Längsende (29) des ersten Zug-/Schubteils (3) bewegbar ist oder sind.

11. Biopsiezange (1) gemäß einem oder mehreren der vorangehenden Ansprüche, **dadurch gekennzeichnet, dass** die flexible Ummantelung (4) aus Kunststoff, insbesondere aus Polyetheretherketon (PEEK) hergestellt ist.

12. Biopsiezange (1) gemäß einem oder mehreren der vorangehenden Ansprüche, **dadurch gekennzeichnet, dass** die Handhabungseinrichtung (6) an ihrem distalen Längsende einen Luer-Anschluss oder einen Luer-Lock-Anschluss (37) zum Anschließen an einen Arbeitskanal eines Endoskops aufweist.

## Claims

1. Biopsy forceps (1), in particular puncture biopsy forceps,
the biopsy forceps (1) comprising a forceps head (2),
the biopsy forceps (1) comprising a first pull/push part (3), on the distal longitudinal end of which the forceps head (2) is attached,
the biopsy forceps (1) comprising flexible sheathing (4) which has a hollow conduit (5) through which the first pull/push part (3) is longitudinally displaceably guided relative to the flexible sheathing (4),
the biopsy forceps (1) comprising a handling device (6) which has a holding device (7) and a first actuation device (8),
the holding device (7) and the first actuation device (8) together forming a first longitudinal guide (9), by means of which the first actuation device (8) is displaceably guided on the holding device (7), relative thereto, in an actuation direction (X),
the flexible sheathing (4) being connected, in particular at its proximal longitudinal end (10), to the holding device (7),
the first pull/push part (3) being connected, in particular at its proximal longitudinal end (11), to the first actuation device (8),
the biopsy forceps (1) comprising a path-limiting device (15), which can be detachably fastened to the first longitudinal guide (9) at positions that can be selected on the basis of the stated actuation direction (X), and which, in the fastened state, forms a stop which limits a displacement path of the first actuation device (8) in the distal direction relative to the holding device (7), the holding device (7) comprising a guide core, the first actuation device (8) comprising a guide sleeve (14), the guide core (13) and the guide sleeve together forming the first longitudinal guide (9), the path-limiting device (15) comprising a sleeve (16) which is pushed onto the guide core (13), the sleeve (16) comprising in its wall a threaded hole (17) into which a clamping screw (18) is screwed, the sleeve (16) being displaceable on the guide core (13) when the clamping screw (18) is loosened and the sleeve (16) being fixed on the guide core (13) when the clamping screw (18) is tightened, **characterised in that** a plurality of markings (22), in particular numbers, which are spaced apart from one another in the actuation direction (X), are applied to the guide core (13), the sleeve comprising a cut out (23) through which the markings (22) can be read.

2. Biopsy forceps (1) according to claim 1, **characterised in that** the forceps head (2) comprises two branches, it being possible to move one branch or both branches between a closed position and an open position.

3. Biopsy forceps (1) according to one or more of the preceding claims, **characterised in that**, if the first actuation device (8) is located, relative to the holding device (7), in a proximal end position determined by a proximal stop, the forceps head (2) is held in the flexible sheathing (4) and **in that**, if the sleeve (16) and the first actuation device (8) are located, relative to the holding device (7), in a distal end position determined by a distal stop of the holding device (7), the forceps head (2) and a longitudinal portion of the first pull/push part (3), which portion is proximally adjacent thereto, projects distally from the distal longitudinal end of the flexible sheathing (4).

4. Biopsy forceps (1) according to one or more of the preceding claims, **characterised in that** the handling device (6) comprises a second actuation device (25) and a guide part (26) which, together with the second actuation device (25), forms a second longitudinal guide (27) and is fastened to or formed on the first actuation device (8), in particular on the proximal end thereof, **in that** the biopsy forceps (1) comprises a second pull/push part (28) which extends in its longitudinal direction as far as the second actuation device (25) so as to be displaceable through a particular hollow conduit of the flexible sheathing (4), the holding device (7), the first actuation device (8) and the guide part (26) and which second pull/push part is fastened at its proximal longitudinal end to the second actuation device (25) and is connected at its distal longitudinal end (29) to the branches (24).

5. Biopsy forceps (1) according to one or more of the preceding claims, **characterised in that** the first pull/push part (3) is formed as a small tube (3') or at least comprises a small tube (3'), **in that** the second pull/push part (28) is formed as a wire (28') or at least comprises one wire (28') and **in that** the second pull/push part (28) is displaceably guided in its longitudinal direction through a hollow conduit (30) of the first pull/push part (3).

6. Biopsy forceps according to one or more of the preceding claims, **characterised in that** one branch or **in that** both branches (24) is/are held on the forceps head (2) so as to be rotatable on a joint (38), **in that** a lever piece (33) is proximally connected to each rotatable branch (24) and is hingedly connected at its proximal end to a lever part (34) in each case, and **in that** each lever part (34) is connected at its proximal end to the distal longitudinal end (29) of the second pull/push part (28).

7. Biopsy forceps (1) according to one or more of the preceding claims, **characterised in that** the proximal side of the first actuation device (8) is connected to the guide part (26) which is formed as a second guide pin, **in that** the second actuation device (25) is in the form of a grip coil (25') and is displaceably pushed onto the second guide pin in an actuation direction (x).

8. Biopsy forceps (1) according to one or more of the preceding claims, **characterised in that** a first branch (24') is longer than the second branch (24") so that, in the closed position, said first branch extends distally further than the second branch (24"), the first branch (24') in particular being tapered at its free longitudinal end to a tip (35).

9. Biopsy forceps (1) according to one or more of the preceding claims, **characterised in that** the first branch (24') comprises a lateral recess (36) which, in the closed position, is geometrically suitable for receiving the second branch (24") in part or completely.

10. Biopsy forceps (1) according to one or more of the preceding claims, **characterised in that** one or both branches (24) is or are held so as to be pivotable on the forceps head (2) and can be moved, relative to the distal longitudinal end (29) of the first pull/push part (3), by means of the second pull/push part (28).

11. Biopsy forceps (1) according to one or more of the preceding claims, **characterised in that** the flexible sheathing (4) is made of plastics material, in particular of polyether ether ketone (PEEK).

12. Biopsy forceps (1) according to one or more of the preceding claims, **characterised in that** the handling device (6) comprises at its distal longitudinal end a Luer connector or a Luer-lock connector (37) for connection to a working conduit of an endoscope.

## Revendications

1. Pince à biopsie (1), en particulier pince à biopsie pour ponction,
la pince à biopsie (1) comprenant une tête de pince (2),
la pince à biopsie (1) comprenant un premier élément de traction/poussée (3) à l'extrémité longitudinale distale de laquelle est fixée la tête de pince (2),
la pince à biopsie (1) comprenant une gaine flexible (4) qui présente un canal creux (5) à travers lequel le premier élément de traction/poussée (3) est guidée de manière déplaçable longitudinalement par rapport à la gaine flexible (4),
la pince à biopsie (1) comprenant un dispositif de manipulation (6) qui comporte un dispositif ou moyen de prise (7) et un premier dispositif ou moyen d'actionnement (8),
le dispositif ou moyen de prise (7) et le premier dispositif ou moyen d'actionnement (8) forment ensemble un premier guidage longitudinal (9) au moyen duquel le premier dispositif ou moyen d'actionnement (8) est guidé sur le dispositif ou moyen de prise (7) de manière déplaçable par rapport à celui-ci dans une direction d'actionnement (X),
la gaine flexible (4) étant liée, en particulier à son extrémité longitudinale proximale (10), au dispositif ou moyen de prise (7),
le premier élément de traction/poussée (3) étant reliée, en particulier à son extrémité longitudinale proximale (11), au premier dispositif ou moyen d'actionnement (8),
la pince à biopsie (1) comprenant un dispositif de limitation de déplacement (15) qui peut être fixé de manière amovible au premier guide longitudinal (9) dans des positions sélectionnables par rapport à ladite direction d'actionnement (X) et qui, à l'état fixé, constitue une butée qui limite un trajet de déplacement du premier dispositif ou moyen d'actionnement (8) par rapport au dispositif ou moyen de prise (7) dans une direction distale, le dispositif ou moyen de prise (7) comprenant un noyau de guidage, le premier dispositif ou moyen d'actionnement (8) comprenant un manchon de guidage (14), le noyau de guidage (13) et le manchon de guidage formant ensemble le premier guidage longitudinal (9), le dispositif de limitation de trajet (15) comportant un manchon (16) qui est monté sur le noyau de guidage (13), le manchon (16) comportant dans sa paroi un perçage ou alésage fileté (17) dans lequel est vissée une vis de serrage (18), le manchon (16) étant déplaçable sur le noyau de guidage (13) lorsque la vis de serrage (18) est desserrée et le manchon (16) étant fixé au noyau de guidage (13) lorsque la vis de serrage (18) est serrée,
**caractérisée en ce que** sont prévus sur le noyau de guidage (13) une pluralité de marquages (22), en particulier des nombres, espacés les uns des autres dans la direction d'actionnement (X), le manchon ayant un évidement (23) à travers lequel des marquages (22) peuvent être lus.

2. Pince à biopsie (1) selon la revendication 1, **caractérisée en ce que** la tête de pince (2) présente deux branches, une branche ou les deux étant mobile(s) entre une position fermée et une position ouverte.

3. Pince à biopsie (1) selon une ou plusieurs des revendications précédentes, **caractérisée en ce que**, lorsque le premier dispositif ou moyen d'actionnement (8) est dans une position extrémale proximale par rapport au dispositif ou moyen de prise (7) qui est déterminée par une butée proximale, la tête de pince (2) est reçue dans la gaine souple (4), et **en ce que**, lorsque le manchon (16) et le premier dispositif ou moyen d'actionnement (8) sont situés par rapport au dispositif ou moyen de prise (7) dans une position d'extrémité distale déterminée par une butée distale du dispositif ou moyen de prise (7), la tête de pince (2) et une partie longitudinale du premier élément de traction/poussée (3) qui lui est proximalement adjacent dépassent distalement de l'extrémité longitudinale distale de la gaine souple (4).

4. Pince à biopsie (1) selon une ou plusieurs des revendications précédentes, **caractérisée en ce que** le dispositif de manipulation (6) présente un deuxième dispositif ou moyen d'actionnement (25) et un élément de guidage (26) qui forme un deuxième guidage longitudinal (27) avec le deuxième dispositif ou moyen d'actionnement (25) et qui est fixé au ou formé sur le premier dispositif ou moyen d'actionnement (8), en particulier à son extrémité proximale, et que la pince à biopsie (1) présente un deuxième élément de traction/poussée (28) qui s'étend de manière déplaçable dans sa direction longitudinale à travers un canal creux respectif de la gaine flexible (4), du dispositif ou moyen de prise (7), du premier dispositif ou moyen d'actionnement (8) et de l'élément de guidage (26) jusqu'au deuxième dispositif ou moyen d'actionnement (25), qui est fixé à son extrémité longitudinale proximale au deuxième dispositif ou moyen d'actionnement (25) et qui est lié aux branches (24) par son extrémité longitudinale distale (29).

5. Pince à biopsie (1) selon une ou plusieurs des revendications précédentes, **caractérisée en ce que** le premier élément de traction/poussée (3) est réalisé sous la forme d'un tube (3') ou comporte au moins un tube (3'), **en ce que** le deuxième élément de traction/poussée (28) est réalisé sous la forme d'un fil (28') ou comporte au moins un fil (28') et **en ce que** le deuxième élément de traction/poussée (28) est guidé à travers un canal creux (30) du premier élément de traction/poussée (3) de manière déplaçable dans sa direction longitudinale.

6. Pince à biopsie selon une ou plusieurs des revendications précédentes, **caractérisée en ce qu'**une ou les deux branches (24) sont maintenues de manière pivotante sur une articulation (38) de la tête de pince (2), **en ce que** chaque branche pivotante (24) se poursuit proximalement par un élément de levier (33) relié chacun de manière pivotante à son extrémité proximale à une partie de levier (34) et **en ce que** chaque partie de levier (34) est reliée à son extrémité proximale à l'extrémité longitudinale distale (29) du deuxième élément de traction/poussée (28).

7. Pince à biopsie (1) selon une ou plusieurs des revendications précédentes, **caractérisée en ce que** le premier dispositif ou moyen d'actionnement (8) est relié sur son côté proximal à l'élément de guidage (26) conçu comme deuxième broche de guidage, **en ce que** le deuxième dispositif ou moyen d'actionnement (25) est conçu comme bobine de prise (25') et est monté de manière coulissante sur la deuxième broche de guidage dans une direction d'actionnement (x).

8. Pince à biopsie (1) selon une ou plusieurs des revendications précédentes, **caractérisée en ce qu'**une première branche (24') est plus longue que la deuxième branche (24") de manière qu'en position fermée, elle s'étende distalement plus loin que la deuxième branche (24"), dans laquelle est prévu en particulier que la première branche (24') se rétrécisse en une pointe (35) à son extrémité longitudinale libre.

9. Pince à biopsie (1) selon une ou plusieurs des revendications précédentes, **caractérisée en ce que** la première branche (24') présente un évidement latéral (36) géométriquement adapté à la réception partielle ou complète de la deuxième branche (24") en position fermée.

10. Pince à biopsie (1) selon une ou plusieurs des revendications précédentes, **caractérisée en ce qu'**une ou les deux branches (24) est/sont maintenue(s) de manière pivotante sur la tête de pince (2) et est/sont déplaçable(s) par rapport à l'extrémité longitudinale distale (29) du premier élément de traction/poussée (3) au moyen du deuxième élément de traction/poussée (28).

11. Pince à biopsie (1) selon une ou plusieurs des revendications précédentes, **caractérisée en ce que** la gaine souple (4) est en matière plastique, en particulier en polyétheréthercétone (PEEK).

12. Pince à biopsie (1) selon une ou plusieurs des revendications précédentes, **caractérisée en ce que** le dispositif ou moyen de prise (6) présente à son extrémité longitudinale distale un connecteur Luer ou un connecteur Luer Lock (37) pour la connexion à un canal de travail d'un endoscope.
